# EUROPEAN PATENT SPECIFICATION

(11) **EP 1 613 204 B1**
(45) Date of publication and mention of the grant of the patent: **19.08.2009**
(21) Application number: 04758954.4
(22) Date of filing: 26.03.2004
(51) Int. Cl.: G01N 33/68, C07K 16/18

(54) **ASSESSING NEURONAL DAMAGE FROM BLOOD SAMPLES**
BEURTEILUNG VON NERVENSCHÄDEN ANHAND VON BLUTPROBEN
EVALUATION DES LESIONS NEURONALES A PARTIR DE PRELEVEMENTS SANGUINS

(30) Priority: 31.03.2003 US 459286 P
(43) Date of publication of application: 11.01.2006
(73) Proprietor: University of Florida Research Foundation, Inc., Gainesville, FL 32611 (US)
(72) Inventor: SHAW, Gerry, Gainesville, FL 32608 (US); PIKE, Brian, R., Derwood, MD 20855 (US); TULI, Sonal, S., Gainesville, FL 32608 (US)
(74) Representative: Ferreccio, Rinaldo
(86) International application number: PCT/US2004/009114
(87) International publication number: WO 2004/091379

(56) References cited:
- PETZOLD A ET AL: "A specific ELISA for measuring neurofilament heavy chain phosphoforms" JOURNAL OF IMMUNOLOGICAL METHODS, ELSEVIER SCIENCE PUBLISHERS B.V.,AMSTERDAM, NL, vol. 278, no. 1-2, July 2003 (2003-07), pages 179-190, XP004453174 ISSN: 0022-1759
- HU Y-Y ET AL: "Elevated levels of phosphorylated neurofilament proetins in cerebrospinal fluid of Alzheimer disease patients" NEUROSCIENCE LETTERS, LIMERICK, IE, vol. 320, no. 3, 8 March 2002 (2002-03-08), pages 156-160, XP002992789 ISSN: 0304-3940
- HASHIMOTO RYOTA ET AL: "Quantitative analysis of neurofilament proteins in Alzheimer brain by enzyme linked immunosorbent assay system" PSYCHIATRY AND CLINICAL NEUROSCIENCES, vol. 53, no. 5, October 1999 (1999-10), pages 587-591, XP002409716 ISSN: 1323-1316
- SKOUEN J S ET AL: "Protein markers in cerebrospinal fluid in experimental nerve root injury. A study of slow-onset chronic compression effects or the biochemical effects of nucleus pulposus on sacral nerve roots." SPINE. 1 NOV 1999, vol. 24, no. 21, 1 November 1999 (1999-11-01), pages 2195-2200, XP009075589 ISSN: 0362-2436
- HU Y. ET AL.: 'Elevated levels of phosphorylated neurofilament proetins in cerebrospinal fluid of Alzheimer disease patients.' NEUROSCIENCE LETTERS vol. 320, no. 3, 08 March 2002, pages 156 - 160, XP002992789
- 'Current Protocols in Molecular Biology', 1991 article HORNBECK P. ET AL.: 'Enzyme-linked immunosorbent assays (ELISA)', pages 11 - 22, XP008055711
- NYLEN R. ET AL.: 'Cerebrospinal fluid neurofilament and glial acidic protein in patients with cerebral vasculitis.' JOURNAL OF NEUROSCIENCE RESEARCH vol. 67, no. 6, 2002, pages 844 - 851, XP008055710
- 1992, PIERCE CATALOG pages 7 - 37
- POSMANTUR R. ET AL.: 'Neurofilament 68 and neurofilament 200 protein levels decease after traumatic brain injury.' JOURNAL OF NEUROTRAUMA vol. 11, no. 5, October 1994, pages 533 - 545, XP008055655

## Description

### STATEMENT AS TO FEDERALLY SPONSORED RESEARCH

This invention was made with United States government support under grant number DAMD17-01-1-0765 awarded by the United States Army, and grant number AA012151-02 awarded by the National Institutes of Health. The United States government may have certain rights in the invention.

### FIELD OF THE INVENTION

The invention relates generally to the fields of biology and medicine. More particularly, the invention relates to detecting damage to neuronal cells by analyzing a biological sample for neurofilament (NF) H protein released from damaged neurons.

### BACKGROUND

In recent years much interest has been focused on the detection of specific marker proteins in blood to rapidly diagnose various kinds of damage and disease states. Such so-called biomarkers, when studied in detail, have the potential to provide quick and simple diagnosis of a variety of damage and disease states. For example, it has been known for many years that the presence of tissue polypeptide antigen (TPA) in human serum is a useful biomarker for several forms of carcinoma, and the level of TPA expression is negatively correlated with cancer prognosis. TPA was initially identified by raising antisera against the insoluble residues of extracted human tumors, and the assumption from the early work was that the components of TPA would be tumor-specific proteins (Bjorklund, B, Antibiot. Chemother. 22:16-31, 1978). However later studies indicated that TPA was actually a complex of partially degraded keratins 8, 18 and 19, which are abundant components of the cytoskeleton of normal differentiating epithelia cells as well as of carcinoma cells (Weber et al., Embo. J. 3:2707-2714, 1984). Apparently the rapidly dividing carcinoma cells release some of their cytoplasmic components into the serum where they are somewhat resistant to serum proteases and so can be detected by appropriate immunological tests. Individuals with carcinomas of the appropriate type therefore have much larger amounts of these circulating protein fragments than do normal individuals. Since the level of TPA expression in serum accurately reflects the carcinoma cell load, TPA determinations have both diagnostic and prognostic value. Another example of this kind of approach is the monitoring of myocardial infarction, in which levels of cardiac creatine kinase and cardiac troponin I are measured. The serum content of these proteins, released from damaged cardiac cells, provides medically useful information bearing on the size of the infarction which has prognostic value. These kinds of finding and many others establish the principle that normal proteins of cells may be expressed at much higher levels in serum in certain specific kinds of damage and disease state, and their immunological detection may be of diagnostic and prognostic use.

Although diseases associated with neuronal injury are a major health concern worldwide, a truly reliable and convenient specific biomarker of neuronal injury has not been found, even though such a marker has great scientific and potential clinical usefulness (Ingebrigtsen and Romner, J. Trauma 52:798-808, 2002). A few potential markers of brain injury have been described but all have disadvantages. For example, previous studies have proposed that S100-β, neuron specific enolose (NSE) (Persson et al., Stroke 18:911-918, 1987) and more recently spectrin breakdown products (SBPs, Pike et al., J Cereb Blood Flow Metab 24:98-106, 2004) in biological samples may be useful for measuring brain injury. However neither S100-β nor SBPs, are specific for neuronal or even nervous system damage. Neuron-specific enolase looks more promising, since it is expressed in large amounts only in neurons, but has not been widely used perhaps because NSE is a relatively unstable protein. Microtubule associate protein (MAP) tau has also been proposed as a biomarker of neuronal injury (Zemlan et al., J Neurochem 72:741-750, 1999). However it is not a particularly abundant protein and is also expressed in non-neuronal cells (e.g., reactive astrocytic glial cells (Togo and Dickson, Acta Neuropathol. 104:398-402, 2002)). A need therefore exists for a rapid and reliable diagnostic assay that can be used to conveniently assess neuronal damage. Such an assay would be useful to assess neuronal injury in experimental animals and to monitor the effects of drugs which may be neuroprotective in these animals. Such an assay would be particularly useful if the relevant molecule could be detected in blood rather than cerebro spinal fluid (CSF), since obtaining blood is not only routine in research and medical contexts, but is also much easier, less invasive and less potentially dangerous than obtaining CSF. The potential biomarker would be particularly useful if it could be detected in blood within a few hours of trauma, since this would allow it to be used in the emergency room to monitor human accident victims with potential neuronal injury in the spinal cord or brain. It is difficult to determine how much neuronal injury has occurred in accident victims using current X-ray, CAT scan and MRI technology. The detection and quantitation of a biomarker of neuronal injury may therefore have considerable diagnostic and prognostic value in humans. Hu Y-Y et al. "Elevated levels of phosphorylated neurofilament proteins in cerebrospinal fluid of Alxheimer disease patients", Neuroscience Letters, Limerick, IE, vol. 320, no. 3, 8 march 2002, pp. 156-160, disclose that the levels of non-phosphorylated NF-H/M and NF-L are significantly higher in patients with Alzheimer disease and vascular dementia. They further disclose that an increased level of phosphorylated NF-H/M and NF-H alone is also linked to Alzheimer disease and they used a sandwich ELISA for NF subunits, incuding NF-H, in cerebrospinal fluid. Hashimoto Ryota et al., "Quantitative analysis of neurofilament proteins in Alzheimer brain by enzyme linked immunosorbent assay system", Psychiatry and Clinical Neurosciences, vol. 53, no. 5, October 1999, pp. 587-591, describe the detection of two NF subunits (-L and -H) in Alzheimer brain and report that phosphorylated NF-H is increased in the brains. Skuoen J S et al., "Protein markers in cerebrospinal fluid in experimental nerve root injury. A study of slow-onset chronic compression effects or the biochemical effects of nucleus pulposus on sacral nerve roots", Spine, 1 Nov. 1999, vol. 24, no. 21, 1 November 1999 (1999-11-01), pp. 2195-2200, report that a sandwich ELISA was used to demonstrate that NF-L levels are elevated in cerebrospinal fluid after traumatic neuronalinjury (nerve root compression).

### SUMMARY

The application relates to the discovery that injury to central nervous system (CNS) tissue such as spinal cord or brain in an experimental animal leads to the leakage of proteins originating from NF that can be detected in biological fluids such as blood and CSF of the animal. The presence of these NF-derived proteins can be detected using assays utilizing antibodies that specifically bind particular NFDPs (neurofilament - derived proteins and peptides). Because NF expression is absolutely restricted to neurons, measurement of NFDPs provides a way to specifically and unambiguously detect neuron damage.

NF are composed predominantly of three subunit proteins, namely NF-L, NF-M, NF-H, with smaller amounts of two further proteins, α-internexin and peripherin (Shaw, 1998 Neurofilaments. New York: Springer). When neurons are damaged NF subunits, normally found in stable 10nm diameter filaments, are broken down to soluble components under the influence of various endogenous enzymes, such as the calpains, cathepsins, caspases and others. These enzymes produce a family of soluble NFDPs. NFDPs are soluble and diffusible proteins derived from assembled NF, and may be either fully intact NF subunit proteins or proteolytically processed fragments of NF subunits.

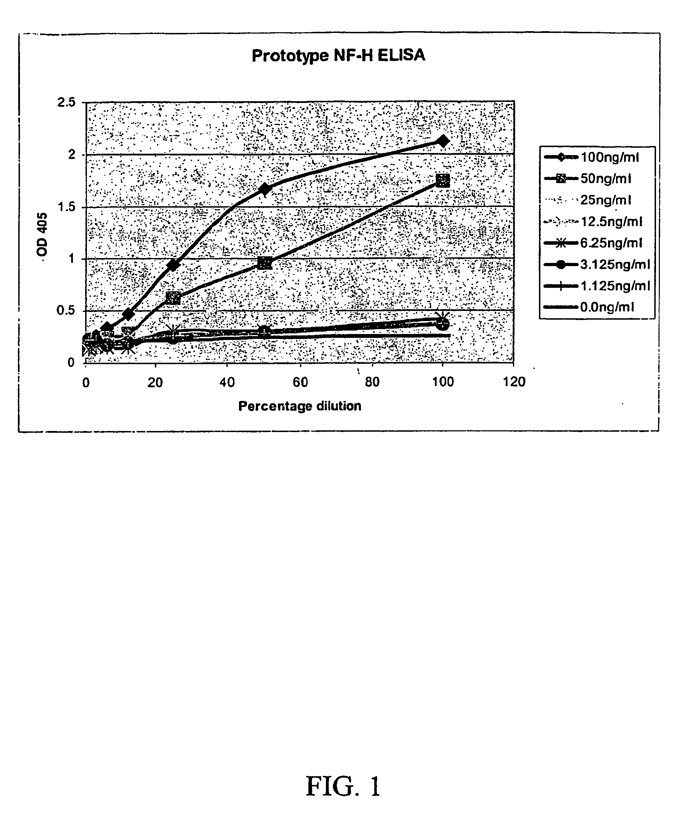

The NF subunit most resistant to proteases is NF-H and this, coupled with some unusual protein chemical and immunological properties of this molecule suggested that this was the most likely to be easily detected in blood, CSF and other bodily fluids following neuronal injury. Based on the foregoing a prototype enzyme-linked immunosorbent assay (ELISA) capture assay was developed. Current version of this assay can reliably detect NF-H in small 50µl volumes in quantities as low as 50pg (equivalent to 1ng/ml or 1µg/L, see figure 1). The prototype assay was to examine NF-H immunoreactivity in control rat blood and in the blood of rats which had been subjected to various different experimental neuronal injuries. No NF-H immunoreactivity could be detected in control blood, but up to 60µg/L NF-H immunoreactivity was detected in the blood of rats given experimental spinal cord injuries. Rats given experimental traumatic brain injuries also showed reproducible but rather lower NF-H signals in blood. Various other neuronal injury paradigms have revealed reproducible NF-H signals in the blood. In summary, a whole series of experiments show that NF-H can be detected by an appropriate antibody based assay in blood of animals which have received experimental neuronal injuries. The NF-H signal could be readily detected not only in blood, but also in sera following clotting of blood and also in plasma, showing that the signal was present in the soluble fraction of blood and not associated with red blood cells or other cellular components.

Specifically, rats were given spinal cord hemisection at the thoracic levels T11, T12 levels. This system is used to model knife and bullet wounds to the CNS. Samples of blood taken from the site of the cut injury showed very high levels of NF-H expression (>80µg/L), showing that NF-H is released in easily detectable amounts immediately following this kind of neuronal injury. Samples of blood taken from the tail at 2, 8, 16 and 24 hours, all revealed small but reproducible NF-H signals, with stronger signals at later time points. These findings establish the principle that NF-H is found in blood in the hours following neuronal injury in readily measurable amounts. Interestingly the levels of NF-H in this series of experiments plateau at about 24 hours, and then go to even higher levels peaking between 3 and 5 days following injury, returning to control levels by about 9 days post injury. This second peak of immunoreactivity is thought to correspond to the secondary death of neurons, and this assay is thought to provide a unique method of measuring this phenomena. This experiment has been performed on a series of animals, all of which produced very similar time courses and degrees of NF-H signal, suggesting that the response of the animal to the injury and the NF-H detection are both reliable and reproducible. Another series of experiments show that experimental spinal cord contusion injuries in rats produce a quantitatively and qualitatively similar NF-H response. Contusion was produced using a standardized weight drop apparatus, and this model system is used to model human crush and impact lesions of the spinal cord. Finally studies of rats subjected to experimental traumatic brain injury, also using a weight drop apparatus, show a measurable but smaller NF-H signal in blood compared to those subjected to spinal cord injury. In summary, blood NF-H levels are able to reliably detect a variety of different kinds of central nervous system neuronal injury.

The ELISA assay described herein is rapid, currently performed in slightly more than three hours, and non-invasive, requiring only a drop of blood. The assay works with fresh blood, serum obtained following clotting at room temperature or plasma obtained by centrifugation. For uniformity, the assay was standardized on plasma which was obtained from fresh blood by centrifugation at 14,000g for 10 minutes at room temperature, 10µl samples from experimental rats was routinely used though greater sensitivity could no doubt be obtained with larger samples. The NF-H signal is quite stable and can be detected without apparent diminution following several cycles of freezing and thawing of blood, serum or plasma, or following several hours at room temperature. This means that the assay is likely to be robust in practice. The assay can be used in animal studies aimed at quantitating neuronal injury and assessing the effectiveness of drugs designed to combat neuronal death. A robust and rapid assay of neuronal injury also has great potential for use on human spinal cord and brain injury patients in the emergency room.

Accordingly, the invention features a method of detecting a neuronal injury in a subject according to claim 1. It can also involve performing an immunoassay selected from the group consisting of immunoblotting, ELISA, radioimmunoassay, immunodiffusion and immunoprecipitation.

The invention also features the use of a kit for detecting and quantitating neuronal injury in a subject according to claims 4 and 5.

As used herein, "bind," "binds," or "interacts with" means that one molecule recognizes and adheres to a particular second molecule in a sample, but does not substantially recognize or adhere to other structurally unrelated molecules in the sample. Generally, a first molecule that "specifically binds" a second molecule has a binding affinity greater than 10⁵ to 10⁶ moles/liter for that second molecule.

The term "blood," as used herein, means the blood derivatives plasma and serum.

By reference to an "antibody that specifically binds" to another molecule is meant an antibody that binds the other molecule, and displays no substantial binding to other naturally occurring proteins other than those sharing the same antigenic determinants as the other molecule. The term "antibody" includes polyclonal and monoclonal antibodies as well as antibody fragments or portions of immunoglobulin molecules that can specifically bind the same antigen as the intact antibody molecule.

The term "subject," as used herein, means a human or non-human animal, including but not limited to mammals such as a dog, cat, horse, cow, pig, rabbit, guinea pig, sheep, goat, primate, rat, and mouse. Since the immunogenic regions of NF-H are well conserved across higher vertebrate species, the current NF-H assay is expected to work on avian and reptilian subjects also. By similar reasoning, assays based on the detection of other NFDPs are also likely to work on all higher vertebrate species.

Unless otherwise defined, all technical terms used herein have the same meaning as commonly understood by one of ordinary skill in the art to which this invention belongs. Although methods and materials similar or equivalent to those described herein can be used in the practice or testing of the present invention, suitable methods and materials are described below. In the case of conflict, the present specification, including definitions, will control. In addition, the particular embodiments discussed below are illustrative only and not intended to be limiting.

### BRIEF DESCRIPTION OF THE DRAWINGS

FIG. 1 is a graph showing the results of BLISA with variable amounts of pure NF-H, illustrating the sensitivity of the prototype assay. The ordinate plots the serial dilution series of a typical FLISA plate, such that the first well is undiluted (100%, to the right), the second is diluted 50%, the third 25% and so on. A straight line indicates a linear and hence quantifiable response. Amounts as low as 50pg of NF-H can be readily and reproducibly detected in small (50µl) samples.

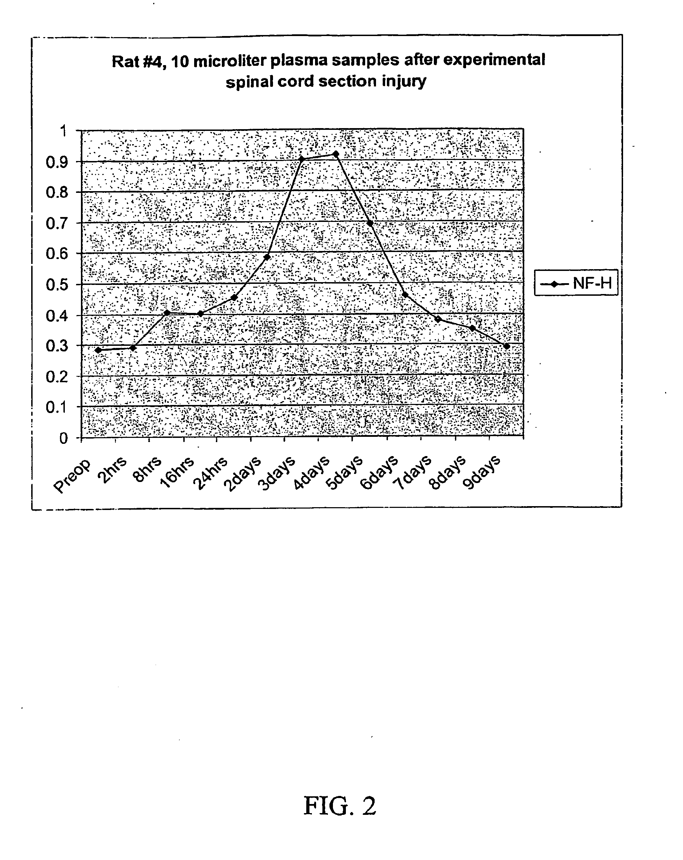

FIG. 2 is a graph showing results from ELISAs performed to determine NF-H concentration in a set of 10µl plasma samples taken at the indicated time from a single animal which had been given an experimental spinal cord hemisection. Note the marked increase in NF-H detectable in the first few hours after injury, and the even greater peak seen after 3-4 days.

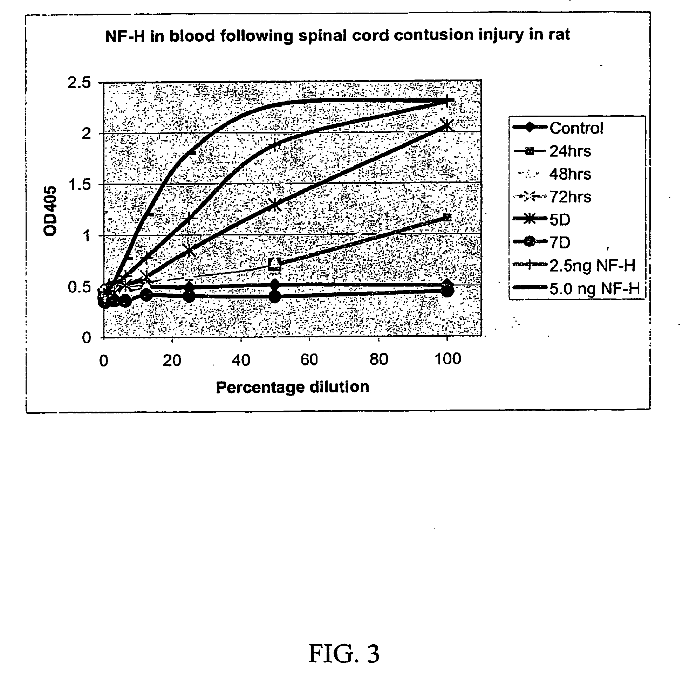

FIG. 3 is a graph showing NF-H immunoreactivity in rat serum following experimental spinal cord contusion injury. 50µl samples of blood were allowed to clot and serum was taken for the ELISA assay. Levels of NF-H increase up to 3-5 days, then decline back to baseline by 7 days.

### DETAILED DESCRIPTION

The invention relates to methods for detecting NF-H in a sample of blood serum or plasma to assess neuronal injury. The below described preferred embodiments illustrate adaptations of these methods. Nonetheless, from the description of these embodiments, other aspects of the invention can be made and/or practiced based on the description provided below.

### Biological Methods

Methods involving conventional biological techniques are described herein. Such techniques are generally known in the art and are described in detail in methodology treatises such as Molecular Cloning: A Laboratory Manual, 3nd ed., voL 1-3, ed. Sambrook et aL, Cold Spring Harbor Laboratory Press, Cold Spring Harbor, N.Y., 2001; and Current Protocols in Molecular Biology, ed. Ausubel et al., Greene Publishing and Wiley-Interscience, New York, 1992 (with periodic updates). Immunological methods (e.g., preparation of antigen-specific antibodies, immunoprecipitation, and immunoblotting) are described, e.g., in Current Protocols in Immunology, ed. Coligan et al., John Wiley & Sons, New York, 1991; and Methods of Immunological Analysis, ed. Masseyeff et al., John Wiley & Sons, New York, 1992.

### NFDPs

NFDPs are generated by the enzymatic digestion of NFs by activated proteases. NFs are the major structural components of neurons and belong to the 10nm diameter or intermediate filament protein family. NFs are composed of the major subunits NF-L, NF-M and NF-H, with certain types of neuron containing smaller amounts of two further subunits, peripherin and α-intemexin. The other members of the 10nm diameter or intermediate filament protein family include the keratins found in epithelial cells, glial fibrillary acidic protein (GFAP) characteristic of astrocytic cells, desmin found in muscle and endothelial cells, vimentin found in many cell types and several less well known proteins. This family of proteins has several interesting properties. First, they are expressed in well defined, specific cell subtype expression patterns. This means, for example, that antibodies to NFs can be used to unequivocally identify cells as being neuronal in origin, and GFAP antibodies are used as the gold standard for the identification of astrocytes. Second, the different intermediate filament proteins and their subunits are extremely abundant components of many cells, and in many large neurons NF subunits may represent several percent of the total amount of protein. Third, NF subunits and the subunits of other 10nm filament are long lived and stable components of the cell which therefore must be rather resistant to normal cellular proteolytic mechanisms. These properties make the 10nm or intermediate filament proteins in general and the neurofilament molecules in particular excellent targets for the development of diagnostic kits aimed at analyzing cell type specific damage.

The large neurons of the mature nervous system are particularly rich in NF-L, NF-M and NF-H. The three polypeptides are each complex multidomain proteins which in the cases of NF-M and NF-H are unusually heavily phosphorylated. When the nervous system is damaged, neuronal cells die either apoptotically or necrotically and are expected to release their contents into the surrounding tissues, the blood and the cerebrospinal fluid. This material is expected to be partially proteolysed, since both apoptosis and necrosis result in the activation of a series of proteolytic enzymes. However NF-H is both highly immunogenic and resistant to proteases, so that intact NF-H or fragments derived from NF-H are likely to be detectable in bodily fluids following release from damaged neurons. As shown above, NF-H can be detected in blood in large amounts at the site of a lesion, in blood as little as two hours after this injury, and in blood over the several days following neuronal injury.

Because NFs are found only in neuronal cells, this approach has a considerable advantage over other methods. Previous workers have used S100-β and spectrin breakdown products which can be detected in blood and in CSF as markers of brain injury. However, both of these proteins are found not only in neurons but also glia, endothelia, and many other types of cells and are not specific to cells within the CNS. Therefore, a test based on NF detection provides much more refined information and offers greater scientific and clinical value since it reflects solely damage to neurons. As noted above MAP-tau and neuron specific enolase are other potential markers of neuronal injury which also have specific disadvantages. A NF detection system is also likely to provide greater sensitivity since NF are thought to be the most abundant neuronal-specific component, and are particularly heavily expressed in large species such as notably human.

### Detecting Neuronal Injuries

The invention provides a method for detecting a neuronal injury in a subject according to claim 1.

The step of providing a biological sample derived from the subject can be performed by conventional medical techniques. A biological sample can be from any site in the body of the subject. While NF-H is expected to accumulate in CSF following neuronal injury, and could be assayed there, a great advantage of the present method is that an adequate signal can be detected in blood. Blood is much more easily obtained than CSF, and is routinely taken from experimental animals and from human patients in the emergency room. No extra specific steps are therefore needed beyond the availability of an appropriate kit to detect NF-H.

Suitable subjects for use in the invention can be any animal species expressing NF which can be detected with our assay system. The subject can therefore be any mammal such as dog, cat, horse, cow, pig, rabbit, guinea pig, sheep, goat, primate, rat, or mouse. It is expected that this assay will work at least on avian and reptilian species, if not also amphibian and fish. A preferred subject for the methods of the invention is a human being. Particularly preferred are subjects suspected of having or at risk for developing traumatic or non-traumatic neuronal injuries, such as victims of neuronal injury caused by traumatic insults (e.g., gunshot wounds, automobile accidents, sports accidents), ischemic events (e.g., stroke, cerebral hemorrhage, cardiac arrest) and neurodegenerative disorders (e.g., Alzheimer's and Parkinson's diseases).

The step of detecting the presence of NF-H in a sample can be performed in a variety of different ways. Numerous suitable techniques are known for analyzing the presence of protein. For example, proteins and specific breakdown products of the same proteins can be detected using immunological techniques, e.g., using antibodies that specifically bind the protein and/or its breakdown products (e.g., NFs, their subunits and breakdown products produced by specific proteases) in immunoassays such as immunoblotting (e.g., Western blotting), ELISA, radioimmunoassay (RIA), immunofluorescence or immunohistochemical staining and analysis, and similar techniques. Suitable methods for detecting NF-H are described below; nonetheless, other suitable methods might also be employed.

Any antibody that binds to NF-H is suitable for use in the invention. In a preferred embodiment, a single antibody can be used to concurrently or independently detect a specific NF-H.

### Kits

The invention includes the use of a kit according to claim 4 and 5. The presence of the detection antibody is visualized and quantified by detection agents such as enzyme-linked antibodies reactive with the detection antibody. The presence of the enzyme linked antibody is detected using chromogenic substrate molecules appropriate for the enzyme. Quantitation of the signal can then be performed by optical density measurements at the wavelength optimum for the particular chromagen. More complex approaches utilize surface plasmon resonance, fluorescence resonance energy transfer or other techniques which involve the use of specialized equipment to assay binding may have advantages in terms of quantifying binding and for high-throughput applications.

In developing the invention, a series of specific polyclonal antibodies to NF subunits were made in rabbit and chicken, and certain monoclonal antibodies were made in mouse. In the prototype ELISA assay described here we used a very high titre chicken polyclonal antibody to NF-H in the capture mode. This was affinity purified on pure NF-H, and coated onto ELISA plates using standard methods: The detection antibody was a rabbit polyclonal antibody which was also affinity purified in the same way. The combination of two polyclonal antibodies made in two different species gives unusual sensitivity to this assay. Other antibodies that specifically bind additional particular NFDPs will be assessed for utility in future. Such kits would include reagents to detect NF-M, NF-L, α-internexin and peripherin. More advanced and automated kits use protein microarrays based on the same antibody reagents. Such arrays could be used in both basic research and clinical (e.g., emergency room) applications. Additionally, a colorimetric filter-based assay using specific immobilized antibodies is within the invention.

### EXAMPLES

### Example 1 - Neurofilament Subunit NF-H As A Robust Serum

### Biomarker Of Neuronal Injury

### Materials and Methods

Development of NF-H specific antibodies: Since bovine tissues can be obtained relatively easily and since the bovine NF-H molecule is immunologically and protein chemically similar to that of humans and other species, we used bovine NF-H to prepare antibodies reactive with NF-H. Bovine spinal cord tissue was obtained from a local slaughter house, transported on ice, desheathed of meninges and stored at -70°C. Neurofilament rich gels were prepared essentially as described by Delacourte et al. (Delacourte et al., Biochem J 191:543-546, 1980). Briefly, ∼250g of the bovine spinal cord material was thawed out and homogenized in a blade type homogenizer in MES Buffer (0.1M MES, 1mM EGTA, 0.5mM MgCl₂ pH=6.5, plus 0.2mM PMSF). The homogenate was filtered through cheese cloth and centrifuged at 14,000 rpm/29,000g for one hour at 4°C. The supernatant was then centrifuged at 28,000 rpm/78,000g for 30 minute at 4°C. Glycerol was added to give a final concentration of 20% and the material was incubated for 20 minutes at 37°C. The supernatant was centrifuged at 45,000 rpm/235,000g for 30 minutes at 20°C. Typically about 3g of clear yellowish pellets were collected per preparation. These pellets typically contain about 90% NF-L, NF-M and NF-H, with smaller amounts of GFAP and fodrin/spectrin. This material was dissolved in 6M urea in 10mM phosphate buffer, 1mM EDTA, 0.1% β-mercaptoethanol, pH = 7.5, and applied to a DEAE cellulose column equilibrated in the same buffer. Proteins were eluted using a NaCl gradient from 0M to 0.25M in the same buffer. A single clean NF-H protein band eluted at about 0.05M NaCl, and this material was concentrated to about 1mg/ml and dialyzed against PBS. 250µg of purified NF-H were mixed 1:1 with Freund's complete adjuvant and injected into mice, rabbits and chickens, and then 3 weeks later animals were boosted with 200µg mixed 1:1 with Freunds incomplete adjuvant.

Following two further boosts, two rabbits were exsanguinated and sera collected for affinity purification. For chickens, eggs were taken and IgY-enriched preparations produced by delipidation in organic solvents followed by ammonium sulphate precipitation. The mice were sacrificed and their spleen cells fused with PAI myeloma were processed for hybridoma production using standard methods. The hybridoma were grown in 6 by 24 well dishes, and were screened by ELISA on the immunogen. The NP1 hybridoma was selected for subcloning as it reacted extremely strongly with NF-H in ELISA and also stained neurons in unfixed and paraformaldehyde fixed histological sections. The chicken polyclonal IgY preparation is designated CPCA-NF-H, the rabbit polyclonal serum is designated RPCA-NF-H and the mouse monoclonal is designated MCA-NP1. All three are now obtainable commercially from EnCor Biotechnology Inc. (Alachua, FL). Prior to use in these assays, both the rabbit sera and IgY preps were affinity-purified on purified NF-H coupled to cyanogen bromide activated Sepharose 4B (Sigma). The mouse monoclonal was affinity purified on a Hi-Trap Protein G column (Amersham) following the manufacturers instructions. Eluted antibodies were dialyzed against PBS prior to use in ELISA assays.

Prototype ELISA Assay: To perform ELISA assays, Immulon 4HBX plates, which are standard 96 well format ELISA coated to improve protein binding, were used. Affinity purified chicken antibody to NF-H was quantified by UV absorbance and 100µl amounts were applied at 0.7 µg/ml in 50mM sodium bicarbonate buffer at pH = 9.5. Plates were incubated at 4°C overnight and then the next day blocked for at least 1 hour with 150µl of 5% Carnation non-fat milk in Tris buffered saline (TBS). Then the plates were washed in TBS plus 0.1% Tween 20 (TBS/Tween) using a Biorad ELISA plate washer set at 300µl volume, 4 seconds soak time per cycle, 5 cycles. Plates could then be stored in a humid box at 4°C in 100µ/well TBS/Tween containing 1mM sodium azide, or could be used for assays immediately. To perform an assay 50µl of ELISA incubation buffer (2% Carnation non fat milk, in TBS/Tween) was applied to each well. Up to 50µl of blood or other protein sample was then applied to the A row of each plate, and this material was serially diluted down the dish, allowing the analysis of up to 12 samples per plate.

After 1 hour incubation with shaking at room temperature, the plate was washed several times in TBS/Tween using a Biorad ELISA plate washer as before. Affinity purified rabbit detection antibody to NF-H at about 50µg/ml concentration was added to 10 mls of ELISA incubation buffer per ELISA plate, and 100µl of this solution was applied to each well. After incubation for 1 hour at room temperature with shaking, the plate was again washed in TBS/Tween on the ELISA washer, each well was incubated with 1:2,000 goat anti-rabbit alkaline phosphatase conjugate (Sigma) in ELISA incubation buffer. After another hour incubation at room temperature with shaking, the plates were washed for a final time on the ELISA plate washer as before and developed with 100µl/well of 0.1 M Glycine, 1mM Mg, 1mM Zn at pH = 10.4 containing 1mg/ml p-Nitrophenyl Phosphate (Sigma). After 20 minutes to 1 hour development, the reaction was stopped with 50µl/well of 2M NaOH, and results were quantitated on a Tecan Spectrafluor plus ELISA plate reader using 405nm absorbance.

Animal Experiments: Female Long-Evans rats weighing 230-300 grams were obtained from Harlan (Indianapolis, IN). All surgical procedures were performed under sterile conditions with supplemental heat. Intraperitoneal administration of Nembutal (sodium pentobarbital) at 50-60 mg/kg was used to induce anesthesia. Following either partial T11, T10 laminectomy with the dura mater intact, injury was produced by scalpel hemisection was performed at the T12, T11 spinal level. A small sample of blood was taken from the cut region. The incisions were closed in layers, and animals were allowed to recover in a heated incubator with food and water ad libitum. Bladders were expressed and fluids were administered when required. In the case of the animals treated with the lesion, blood samples were taken by tail bleeding at 2hrs, 8hrs, 16hrs, 24hrs, 2days and every following day out to 11 days. A typical result is shown in Figure 2. ELISA consistently shows a strong peak of NF-H in serum at 3-4 days post-injury. Significantly however, NF-H can be robustly detected at as little as 8 hours post injury, and a weak but reproducible signal was seen at 2 hours postinjury. ELISA assays showed a consistent and marked expression of NF-H in the blood taken from the injury site, actually much higher than the levels seen in sera at later time points. These experiments show that NF-H is immediately released into the blood following nerve injury and can furthermore be consistently and reproducibly detected in the hours and days following experimental nervous system injury.

It is particularly significant that NF-H is detectable at the site of injury and in the few hours following injury. This allows an assay based on these findings to detect neuronal injury in human patients in the emergency room. Detection of serious neuronal injury by other means, such as MRI, X-Rays, CAT scanning etc, is problematic; An assay based on these findings could rapidly detect neuronal injury in an unconscious patient and the level of NF-H detected is very likely to have prognosticative value.

In another set of experiments spinal cord injury was produced using a standardized New York impactor device with a 10g weight falling 25mm. Sham injury animals received a laminectomy and were placed in the injury apparatus but were not injured. The experimentally treated animals were sacrificed at 24, hours, 48 hours, 72 hours, 5 days, 7 days and 6 weeks after injury. Blood was collected and allowed to clot for 1-2 hours at room temperature, and then stored frozen. As shown in Figure 3 strong signals were obtained from serum samples of animals which had spinal cord contusion injuries. The amount of NF-H detected was significant after 24 hours and increased over 48 and 72 hours. At 5 days the level of NF-H was somewhat lower and by 7 days was almost back to background levels. The signals were surprisingly strong and by comparison with standards, the NF-H level in the experimental animals sera was calculated to be in the range from 26µg/L in the 24 hour animals to as much as 66µg/L in the 72 hour post injury animals. Untreated animals were those which did not have mechanical injuries and were typically being sacrificed at the end of experiments performed for reasons irrelevant to these studies, and revealed no detectable NF-H signal. Further controls were sham treated animals, which were anesthetized and had their spinal cords exposed as did the experimental group, but were not subjected to the weight drop paradigm. None of these animals showed any significant NF-H immunoreactivity with the current assay.

Because it would be advantageous to know exactly what form of NF-H is being detected in this assay, the sera from a rat given a spinal cord injury 3 Days previously and which had shown a strong signal in the ELISA assay was subjected to preliminary fractionation. Fractions of serum protein were obtained by ammonium sulphate precipitation. Fractions were assayed using ELISA, and a weak signal was obtained in the first fraction and a much stronger one in the second fraction, while in subsequent fractions the signal was essentially at background levels. The second fraction was therefore subjected to gel filtration on a Superose column (Pharmacia), and fractions were again screened using the ELISA assay. The NF-H immunoreactivity eluted very early in the profile indicative of a molecular weight in the range of 0.5 million Daltons. This indicates that the NF-H signal is at least multimeric and perhaps part of a complex of proteins.

### Example 2 - Variations of the Assay

In addition to the assay described above, there are many possible variations that may be useful in detecting NFDPs in the sera of an animal. For example, the use of avidin-biotin conjugate-based methods may greatly increase the sensitivity of ELISA assays. As another example, modifications such as using higher antibody concentrations and incubations at 37°C rather than room temperature may improve the assay. The development of assays involving rapid colorimetric or other methods which would allow the determination of NF-H serum level in minutes is also envisioned. Such an approach could potentially be useful in the diagnosis of human patients. A kit which detects NF-H in sera using, for example, a simple diffusion and antibody capture procedure run in a filter, as has been developed for other kinds of biomarker found in sera and other fluids, may be particularly useful. This kit could be used to quantitate the degree of neuronal damage in a variety of situations apart from the examples illustrated here. An assay which detects such markers is useful experimentally in animal studies and is expected to be diagnostically useful in humans. In particular, the degree of neuronal injury in spinal cord injury and traumatic brain injury patients is difficult to determine using MRI or by other current imaging methods. An assay based on detection of a readily detectable neuronal protein such as this one could rapidly assess the degree of neuronal damage in such accident victims. Using methods and compositions of the invention, it is expected that the level of NF-H expression can be correlated with a specific degree of neuronal injury and a specific prognosis.

### Other Embodiments

While the above specification contains many specifics, these should not be construed as limitations on the scope of the invention, but rather as examples of preferred embodiments thereof. Many other variations are possible. Accordingly, the scope of the invention should be determined not by the embodiments illustrated, but by the appended claims and their legal equivalents.

## Claims

1. A method of detecting a neuronal injury in a subject, the method comprising the steps of :
(a) contacting a blood, serum or plasma sample obtained from the subject with an antibody that specifically binds to NF-H in the sample;
(b) detecting the presence or amount NF-H in the sample; and
(c) correlating the presence or amount of NF-H in the sample with the neuronal injury.

2. The method of claim 1, wherein the step (b) of detecting the presence or amount of NF-H comprises performing an immunoassay selected from the group consisting of immunoblotting, ELISA, radioimmunoassay, immunodiffusion and immunoprecipitation.

3. The method of claim 2, wherein the step (b) of detecting the presence or amount of NF-H in the sample comprises performing an ELISA.

4. Use of a kit for detecting a neuronal injury in a subject by the method according to any one of the preceding claims, the kit comprising:
(a) a solid substrate;
(b) at least one antibody that binds specifically to NF-H;
(c) an agent for detecting binding of the at least one antibody to NF-H; and
(d) instructions for using the kit to detect neuronal injury in a subject.

5. The use of claim 4, wherein the agent for detecting binding of the at least one antibody to NF-H comprises a chromogenic substrate molecule.

## Patentansprüche

1. Verfahren zum Detektieren einer neuronalen Verletzung eines Subjekts, wobei das Verfahren die Schritte aufweist:
a) in Berührung bringen einer Blut-, Serum- oder Plasmaprobe, welche von dem Subjekt erhalten wurde, mit einem Antikörper, der spezifisch an NF-H in der Probe bindet,
b) Detektieren der Präsenz oder der Menge an NF-H in der Probe, und
c) Korrelieren der Präsenz oder der Menge an NF-H in der Probe mit der neuronalen Verletzung.

2. Verfahren gemäß Anspruch 1, wobei der Schritt b) des Detektierens der Präsenz oder der Menge an NF-H das Ausführen eines Immuntests umfasst, welcher ausgewählt ist aus der Gruppe bestehend aus: Immun-Blotting, ELISA, Radioimmuntest, Immundiffusion, und Immunpräzipitation.

3. Verfahren gemäß Anspruch 2, wobei der Schritt b) des Detektierens der Präsenz oder der Menge an NF-H in der Probe das Ausführen eines ELISA umfasst.

4. Verwendung eines Kits zum Detektieren einer neuronalen Verletzung eines Subjekts durch das Verfahren gemäß einem der vorhergehenden Ansprüche, wobei der Kit aufweist:
a) ein festes Substrat,
b) mindestens einen Antikörper, der spezifisch an NF-H bindet,
c) einen Stoff zum Detektieren des Bindens des mindestens einen Antikörpers an NF-H, und
d) Anweisungen zur Verwendung des Kits um eine neuronale Verletzung eines Subjekts zu detektieren.

5. Verwendung gemäß Anspruch 4, wobei der Stoff zum Detektieren des Bindens des mindestens einen Antikörpers an NF-H ein chromogenes Substratmolekül umfasst.

## Revendications

1. Procédé de détection d'une lésion neuronale chez un patient, procédé comprenant les étapes consistant à :
(a) mettre en contact un échantillon de sang, de sérum ou de plasma prélevé sur le patient avec un anticorps qui se fixe spécifiquement à la NF-H dans l'échantillon ;
(b) détecter la présence ou la quantité de NF-H dans l'échantillon ; et
(c) corréler la présence ou la quantité de NF-H dans l'échantillon à la lésion neuronale.

2. Procédé selon la revendication 1, dans lequel l'étape (b) consistant à détecter la présence ou la quantité de NF-H comprend la réalisation d'un dosage immunologique choisi dans le groupe comprenant « l'immunoblotting », la technique ELISA, le dosage radioimmunologique, l'immunodiffusion et l'immunoprécipitation.

3. Procédé selon la revendication 2, dans lequel l'étape (b) consistant à détecter la présence ou la quantité de NF-H dans l'échantillon comprend la réalisation d'un test ELISA.

4. Utilisation d'un kit pour détecter une lésion neuronale chez un patient avec le procédé selon l'une quelconque des revendications précédentes, le kit comprenant :
(a) un substrat solide ;
(b) au moins un anticorps se fixant spécifiquement à la NF-H ;
(c) un agent pour détecter la liaison du au moins un anticorps à la NF-H ; et
(d) des instructions pour utiliser le kit pour détecter une lésion neuronale chez un patient.

5. Utilisation selon la revendication 4, dans laquelle l'agent pour détecter la liaison du au moins un anticorps à la NF-H comprend une molécule formant substrat chromogène.
